# EUROPEAN PATENT APPLICATION

(11) **EP 3 006 053 A1**
(43) Date of publication of application: **13.04.2016**
(21) Application number: 13885847.7
(22) Date of filing: 27.05.2013
(51) Int. Cl.: A61L 2/20

(54) **MOBILE STERILIZING APPARATUS**

(71) Applicant: Marin Guerrero, Alfonso, 08185 Lliça de Vall Barcelona (ES)
(72) Inventor: Marin Guerrero, Alfonso, 08185 Lliça de Vall Barcelona (ES)
(74) Representative: Morgades y Manonelles, Juan Antonio
(86) International application number: PCT/ES2013/070338
(87) International publication number: WO 2014/191585

(57) **Abstract**

The invention refers to an inside and outside container arrangement comprising interconnected containers, each with individual features and transportable on trucks, trains or other transport vehicles, as they are entirely conventional externally. They comprise a mobile ethylene oxide sterilizing plant that can be set up in any location, as an annexe to any existing factory buildings or installations.

## Description

### Object of the Invention.

More specifically the invention refers to an inside and outside container arrangement comprising interconnected containers, each with individual features and transportable on trucks, trains or other transport vehicles, as they are entirely conventional externally. They comprise a mobile ethylene oxide sterilizing plant that can be set up in any location, as an annexe to any existing factory buildings or installations.

### State of the Art.

Conceptually speaking, sterilization should be differentiated from disinfection, which aims to destroy the pathogenic germs that can be found on the surface of any object or product, from antisepsis, which aims to impede or delay the development of pathogenic and non-pathogenic germs; Of asepsis, which is a collection of rules that intend to impede or delay the growth of pathogenic germs on a certain material, and disinfection, which expels or keeps away or destroys ectoparasites.

The means used for sterilization in the aforementioned operations are often the same, depending on their nature and the choice of type of product to be treated and its specifications. Sterilization used in installations referred to in this invention uses physical means, the most common being ethylene oxide and with the aid of autoclaves.

There are many types and kinds of sterilization installations, but they are static and therefore built of construction material forming part of buildings or factory installations, built with conventional materials and means, however, these installations are not used continuously, and this is why sterilization operations needed by many industries is done outside their factories, outsourced to external suppliers that collect the product and sterilise it, and then protect it so that it cannot again be contaminated, subsequently returning it to the original factory.

There are currently no mobile installations, formed by a collection of containers that can be adapted to existing installations or buildings, quickly and with no need for construction work or supplementary installations to incorporate the design presented here to said installations and/or production plants.

### Scope of the Invention.

The implementation of a sterilization plant in the client's installations within a timeframe of approximately 24 - 48 hours, by means of a layout formed by three or more containers, such as those used for transporting goods by sea or land, which can be easily transported on trailers.

With this design, the client has no need to construct a building, as the containers can be located outside, adjacent to the building and/or existing buildings, so the containers can be grouped in several manners, for example in pyramidal, "L" or "U" shape, according to the client's requirements.

Another of the aims of the invention is to avoid environmental pollution, as there are no CO₂ emissions into the atmosphere.

### Description of the Invention.

The mobile plant is organised by combining (as an example, but not limited to) three or more 40-foot containers, although other dimensions and standards may be used. The first container is modified internally in order to operate as a real "bunker" and contain an autoclave, among other items.

The product to be sterilized arrives at this first container from the corresponding building, by means of an automatic chain conveyor system that pulls pallets of the product inside the container for the processing.

The second container has an airing autoclave, into which the product from the first container enters by means of a chain conveyor system. After the airing time, the product comes out sterile and ready for distribution.

The third container is the service container. It will be divided into two areas; The EEx zone and the non-EEx zone, separate from each other, for which two different inlets have been designed and a partition in the central area. This third container includes all the equipment needed for supplying the autoclave: gas, electricity, air and others needed for the type of product to be treated.

The layout has been designed to operate in different configurations, so as to be able to adapt to different types of buildings and/or installations it must serve, with the sole limitation of needing electrical supply. This layout is not necessarily limited to three containers, as a service container may operate with two or more containers containing an automatic chain conveyor system for the product to be treated.

The functionality of this layout supplies a sterilisation plant for the client without having to invest in an infrastructure, as the containers are installed outside, although in certain buildings it is also possible to place them indoors, as they can be operated from the truck that transports them to their location with a simple crane.

Other details and characteristics shall be shown throughout the description below referring to drawings attached to this report which are shown for illustrative but not limiting purposes only in a drawing of the invention.

### Description of the drawings.

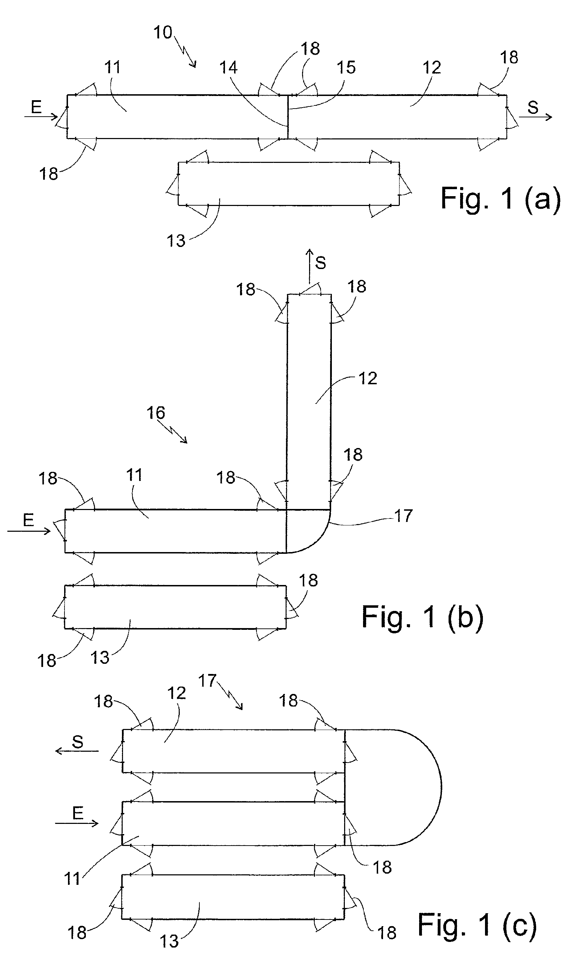

Figure 1, are a schematic diagrams of the layout of the invention in three possible variants, in which (a) the layout (10), presents container (11) aligned with container (12), with the service container (13) next to the other two (11-12), (b) container (12) is located perpendicular to container (11), and the service container (13) next to (11), and (c) the three containers (11-12-13) are placed parallel to each other.

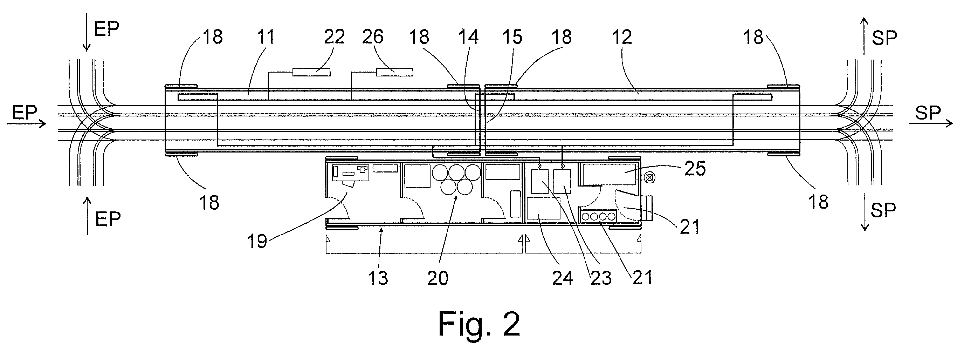

Figure 2 is a detailed top plan view of the layout schematically shown in Figure 1 - (a), in which the elements and equipments integrated inside the containers (11-12-13) may be seen.

Below is a list of the different parts of the invention, which are indicated in the above drawings with their respective numbers; (10) layout, (11) first container, (12) second container, (13) service container, (14) and (15) openings, (16) alternative layout, (17) alternative layout, (18) doors, (19) office, (20) tanks, (21) Weights system, (22) gas circulation pumps, (23) vacuum pumps, (24) steam generator, (25) Scrubber, (26) hydraulic assembly

### Description of an embodiment of the invention.

In one of the preferred embodiments of the invention, as can be seen in Figure 1, the layout (10) can be made in multiple possible configurations (a), (b), and (c), and others in which several container units (11), (12) and (13) can be used, so as to adapt to the different shapes of existing installations and buildings the client may construct in the future, as well as the sterilization capacity (product units) of the installation.

The installation unit is a container (11) for example, with operational access doors (18) that are always kept open, for fitting two or more containers of the first (11) and/or second (12) type, at their openings (14-15) (see Figure 2), opposite these openings once the doors (18) are open, and resting on the side walls of (11-12), being able to connect (11) with (12) when the doors (18) are open with simple bellows type joints for sealing the connection and avoiding the entry of air from outside.

In the configurations (a), (b) and (c) shown for illustrative purposes, but not limited to what is shown in figure 1, so that, for example as can be seen in detail in figure 2, the client's production lines extend to those in the containers (11-12), being attached by simple mechanical means, with the product coming from the client's production building through the entry point "EP" of (11) and exiting through the outlet (12) "SP".

The totally conventional sterilization process is prepared in the service container (13), which is not connected to the other containers (11-12) and is equipped by at least the following elements and/or equipment:
- Control equipment located in the office (19).
- A general electric panel for the whole layout (10).
- Tanks with water and other liquids or gases (20).
- Gas circulation pumps (22) and vacuum pumps (23), one goes to the container's autoclave (11) and the other to the chamber of the container (12).
- A steam generator (24), to supply steam to the autoclave.
- A Scrubber (air cleaner) (25), a washing column for separating one or more of the undesired components in a gas.
- Hydraulic assembly (26).

To avoid any kind of contamination in the sterilisation process carried out in the containers (11-12), and according to other of the characteristics of the invention, the service container (13) where the elements described in the previous section are found, is next to the containers (11-12), but its position regarding them can be any of those displayed in figure 1, and others allowed by the layout, according to the number of containers units (11) and/or (12) comprised in the chosen layout (10).

The containers (11) and (12) in the layout versions shown in figures 1 (b) and (c) will be connected by any means such as purpose-built metal bellows or similar elements, most of which are already available on the market.

The proposed layout (10) will be offered for sale or renting, so that if a client has no longer need for sterilization due to having changed the product, the equipment of said layout (10) can rapidly be transported to another location where it is needed, additionally, it will also be useful if a pharmaceutical company were to need increased sterilization capacity at a certain moment due to overproduction.

Having sufficiently described this invention using the Figures attached, it is easy to understand that any changes judged to be suitable may be made, whenever these changes do not alter of the essence of the invention summarised in the following claims.

## Claims

1. **- Layout for a mobile sterilizing installation** with physical means for using ethylene dioxide to eliminate pathogenic germs or similar organisms, **characterised in that** it is a combination of containers (two or more) inside of which there are conveyors for moving products and sterilising them, these containers being either in line, parallel to each other or at an angle, receiving the sterilization gases at a temperature by means of the necessary conduits, from a service container.

2. **- Layout for a mobile sterilizing installation,** according to the claim one, **characterised in that** the doors of the first and second containers are opposite each other and are sealed from the outside by a bellows.

3. **- Layout for a mobile sterilizing installation,** according to the claim one, **characterised in that** the products to be sterilised enter through "EP" from the first container and exit by "SP" from the second container.

4. **- Layout for a mobile sterilizing installation,** according to the claim one, **characterised in that** the service container is equipped by at least one or more of the following elements and/or equipment:
- Control equipment located in the office.
- A general electric panel for the whole layout.
- Tanks with water and other liquids or gases.
- Gas circulation pumps and vacuum pumps.
- Steam generator.
- A Scrubber, a washing column for separating one or more of the undesired components in a gas.
- Hydraulic assembly.

5. **- Layout for a mobile sterilizing installation,** according to the claim one, **characterised in that** the service container is positioned next to the first and second containers.

6. **- Layout for a mobile sterilizing installation,** according to the claims one and five, **characterised in that**, alternatively, one layout will have the service container immediately after the first container.

7. **- Layout for a mobile sterilizing installation,** according to the claims one and five, **characterised in that**, alternatively, one layout will have the service container immediately after the second container.
